# EUROPEAN PATENT SPECIFICATION

(11) **EP 3 592 341 B1**
(45) Date of publication and mention of the grant of the patent: **01.12.2021**
(21) Application number: 18764435.6
(22) Date of filing: 06.03.2018
(51) Int. Cl.: A61K 31/192, A61K 8/37, A61K 8/02, A61P 17/00, A61Q 17/04, A61Q 19/00, A61K 9/00, A61K 9/107, A61K 31/216, A61Q 19/08, A61K 8/06

(54) **TOPICAL COMPOSITIONS CONTAINING SYNTHETIC ESTERS OF SINAPINIC ACID AND METHODS FOR TREATING KERATIN SURFACES**
TOPISCHE ZUSAMMENSETZUNGEN MIT SYNTHETISCHEN ESTERN VON SINAPINSÄURE UND VERFAHREN ZUR BEHANDLUNG VON KERATINOBERFLÄCHEN
COMPOSITIONS TOPIQUES CONTENANT DES ESTERS SYNTHÉTIQUES D'ACIDE SINAPINIQUE ET PROCÉDÉS DE TRAITEMENT DE SURFACES DE KÉRATINE

(30) Priority: 08.03.2017 US 201762468617 P
(43) Date of publication of application: 15.01.2020
(73) Proprietor: ELC Management LLC, Melville, NY 11747 (US); Symrise AG, 37603 Holzminden (DE)
(72) Inventor: PERNODET, Nadine, Huntington Station, New York 11746 (US); CHEN, Chia-Wen, Eastchester, New York 10709 (US); DONG, Kelly, Merrick New York 11566 (US); HUANG, Jingyu, Brooklyn, New York 11218 (US); KOCH, Oskar, D-37079 Goettingen (DE); BUGDAHN, Nikolas, D-37603 Holzminden (DE)
(74) Representative: Dehns
(86) International application number: PCT/US2018/021193
(87) International publication number: WO 2018/165189

(56) References cited:
- WO-A1-2008/107093
- WO-A2-02/04653
- CN-C- 1 168 725
- US-A1- 2004 258 635
- US-A1- 2004 258 743
- LEWIS A BAKER ET AL: "Observing and understanding the ultrafast photochemistry in small molecules: applications to sunscreens", SCIENCE PROGRESS, SCIENCE REVIEWS 2000 LTD, GB , vol. 99, no. 3 31 August 2016 (2016-08-31), pages 282-311, XP009516882, ISSN: 0036-8504, DOI: 10.3184/003685016X14684992086383 Retrieved from the Internet: URL:http://journals.sagepub.com/doi/full/1 0.3184/003685016X14684992086383 [retrieved on 2020-01-21]
- BAKER, L. A. et al.: "Observing and understanding the ultrafast photochemistry in small molecules: applications to sunscreens", Science Progress, vol. 99, no. 3, September 2016 (2016-09), pages 282-311, XP009516882, DOI: 10.3184/003685016X14684992086383
- ALLAIS, F. et al.: "Straightforward total synthesis of 2-0-feruloyl-L- malate, 2-0-sinapoyl-L-malate and 2-0-5-hydroxyferuloyl-L-malate", Synthesis, vol. 21, 2009, pages 3571-3578, XP055002566, DOI: 10.1055/s-0029-1216983
- SCHIJNDEL, J. V. et al.: "Conversion of syringaldehyde to sinapinic acid through Knoevenagel-Doebner condensation", Open Journal of Physical Chemistry, vol. 6, 2016, pages 101-108, XP055606283,

## Description

### Technical Field

The invention is directed to topical compositions containing a synthetic ester of sinapinic acid that is sinapoyl malate, compositions comprising sinapoyl malate for use in a method for treating skin, and a method for making a topical composition comprising sinapoyl malate.

Plants need sunlight to survive. They have an inherent sunscreen in the form of sinapate esters that protect them from the harmful UVB rays and permit passage of the rays necessary to maintain plant life. Plants genetically engineered so that they are unable to produce sinapate esters suffer crippling damage that leads to stunted growth and withering. Light in the ultraviolet range spans wavelengths ranging from 280 to 400 with UVA light wavelength ranging from 315 to 400 and UVB light ranging from 280 to 315.

Extraction of plant material to obtain sinapate esters is complicated and provides very low yield. The destruction of large numbers of plants to obtain only a nominal amount of extract is simply not cost effective or practical.

A cost effective, efficient synthetic process for preparation of sinapate esters has been discovered. In addition, the sinapate esters prepared according to this synthetic process are found to have a variety of beneficial effects on skin.

US2004/0258635 A1 discloses sunscreen compounds produced by derivatizing milkweed oil with a cinnamic acid selected from ferulic, coumaric, sinapic and o-methylsinapic acid to form esters.

### Summary of the Invention

The invention is directed to topical compositions containing a synthetic ester of sinapinic acid that is sinapoyl malate.

The invention is also directed to a composition for use in a method for treating skin to provide one or more benefits selected from (a) protecting against UV radiation, (b) inhibiting DNA damage in skin cells, (c) inhibiting or reducing skin inflammation, or (d) scavenging free radicals on skin by topically applying the composition, wherein the composition comprises a synthetic ester of sinapinic acid that is sinapoyl malate.

The invention is also directed to a method for making a topical composition comprising synthesizing sinapoyl malate and then formulating the sinapoyl malate into a topical composition. The synthesis of the sinapoyl malate comprises the steps of:
(a) (i) reacting syringaldehyde with acetic acid anhydride and an alkali metal acetate; or (ii) reacting sinapinic acid with acetic acid anhydride,
(b) cleaving the anhydride of the acetyl protected reaction product of (a) by exposing to water and an aliphatic alcohol to form protected sinapinic acid,
(c) esterifying the protected sinapinic acid by reacting with an alkyl protected carboxylic acid ester to form protected sinapoyl malate,
(d) reacting the protected sinapoyl malate with aqueous acid to yield sinapoyl malate.

### Detailed Description of Drawings

Fig. 1: The measured absorbance peaks and valleys for testing of absorbance within the UV range for Sinapoyl malate (SM) and caftaric acid phenethyl ester (CAPE) at concentrations of 1.4 parts per million (ppm) and 14 ppm over times ranging from 0, 1 week, 2 weeks, 4 weeks and at temperatures of 4°, 25° 40°, and 50° C. are set forth in the Table. Sinapoyl malate and CAPE show different absorbance peaks and valleys in the UV range thus demonstrating that the structural difference results in a difference in UV absorption.
Fig. 2: demonstrates the stability of Sinapoyl malate and CAPE absorbance peaks over time and temperature. SM#1 means Sinapoyl malate peak #1, SM#2 peak number 2. CAPE#1 means first CAPE peak. CAPE#2 means second CAPE peak. The top graph shows stability at 1.4 ppm concentration. The bottom graph shows stability at 14 ppm concentration. In both cases it is seen that Sinapoyl malate shows more consistent stability over time. In contrast, CAPE stability wavers up and down and absorbance peaks change or are not maintained.
Fig. 3: demonstrates the cellular viability of normal human epidermal keratinocytes that are not irradiated, or irradiated with 20, 40, and 60 mJ/cm² UVB. Results show that Sinapoyl malate at concentrations ranging from 0.0025 to 0.25% improved cellular health and viability after exposure to varying dosages of UVB radiation.
Fig. 4: graphically demonstrates the effectiveness of Sinapoyl malate in protecting against UV damage both in non-irradiated cells and cells irradiated with UV at 40 mJ/cm² at concentrations ranging from 0.025 to 0.05%.
Fig. 5: demonstrates the effectiveness of Sinapoyl malate as an anti-inflammatory in its ability to inhibit IL1-α and IL1-β in irradiated and non-irradiated cells.
Fig. 6: graphically demonstrates the effectiveness of Sinapoyl malate as an anti-oxidant over concentration ranges from 0.01 to 0.1%.

### Detailed Description

### I. The Synthetic Carboxylic Acid Ester of Sinapinic Acid

The synthetic compound used in the topical compositions and made in the methods of the invention is an ester of sinapinic acid that is sinapoyl malate.

Sinapoyl malate may be synthesized as follows with the designations "S" followed by a numeral defined in Example 1.

The starting material may be either sinapinic acid or syringaldehyde. Sinapinic acid is expensive so it may be more desirable to start with syringaldehyde.

Acetyl protected sinapinic acid S3 may be synthesized using a PERKIN-type reaction as further described in the Examples. Sinapinic acid, S1, may be reacted with acetic acid anhydride in the presence of an appropriate base to form an acetyl protected sinapinic acid. More preferably, syringaldehyde S2 may be reacted with acetic anhydride, sodium acetate, water, and an aliphatic alcohol, preferably methanol to form the acetyl protected sinapinic acid.

The resulting acetyl protected sinapinic acid S3 can be esterified with an alkyl protected malic acid ester S4, which is preferably a dimethyl or diethyl ester, or more preferably a diisopropyl ester using STEGLICH conditions using a carbodiimide coupling reagent such as diisopropyl carbodiimide, or more preferably dicyclohexyl carbodiimide to yield the fully protected sinapoyl malate S5. Complete deprotection may be achieved by hydrolyzing with aqueous acids such as sulfuric acid or hydrochloric acid to yield sinapoyl malate S6.

The topical compositions of the invention may contain 0.01 to 10%, preferably from about 0.05 to 8%, more preferably from about 0.1 to 5% by weight of the synthetic ester of sinapinic acid, with all percentages set forth herein being percentages by weight unless otherwise indicated.

### II. The Topical Compositions

The topical composition may be in the form of a solution, gel, cream, lotion, emulsion or anhydrous product. Preferred is where Sinapoyl malate is formulated into an emulsion containing about 10-90% water and 10-90% oil. The composition may contain other ingredients including but not limited to those set forth herein.

### A. Oils

In the event the compositions of the invention are in emulsion form, the composition will comprise an oil phase. Oily ingredients are desirable for the skin moisturizing and protective properties. Suitable oils include silicones, esters, vegetable oils, synthetic oils, including but not limited to those set forth herein. The oils may be volatile or nonvolatile, and are preferably in the form of a pourable liquid at room temperature. The term "volatile" means that the oil has a measurable vapor pressure, or a vapor pressure of at least about 2 mm of mercury (0.27 kPa) at 20° C. The term "nonvolatile" means that the oil has a vapor pressure of less than about 2 mm of mercury (0.27 kPa) at 20° C. If present, such oils may range from about 0.01 to 85%, preferably from about 0.05 to 80%, more preferably from about 0.1 to 50%.

Suitable volatile oils generally have a viscosity ranging from about 0.5 to 5 centistokes (0.5 to 5 × 10⁻⁶ m²/s) at 25°C. and include linear silicones, cyclic silicones, paraffinic hydrocarbons, or mixtures thereof.

Cyclic and linear volatile silicones are available from various commercial sources including Dow Corning Corporation and General Electric. The Dow Corning linear volatile silicones are sold under the tradenames Dow Corning 244, 245, 344, and 200 fluids. These fluids include hexamethyldisiloxane (viscosity 0.65 centistokes (abbreviated cst) [0.65 × 10⁻⁶ m²/s]), octamethyltrisiloxane (1.0 cst [1.0 × 10⁻⁶ m²/s]), decamethyltetrasiloxane (1.5 cst [1.5 × 10⁻⁶ m²/s]), dodecamethylpentasiloxane (2 cst [2 × 10⁻⁶ m²/s]) and mixtures thereof, with all viscosity measurements being at 25°C.

Suitable branched volatile silicones include alkyl trimethicones such as methyl trimethicone having the general formula: 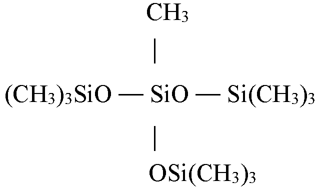 Methyl trimethicone may be purchased from Shin-Etsu Silicones under the tradename TMF-1.5, having a viscosity of 1.5 centistokes (1.5 × 10⁻⁶ m²/s) at 25°C.

A variety of nonvolatile oils are also suitable for use in the compositions of the invention. The nonvolatile oils generally have a viscosity of greater than about 5 to 10 centistokes (greater than 5 to 10 × 10⁻⁶ m²/s) at 25°C., and may range in viscosity up to about 1,000,000 centipoise (1 m²/s) at 25°C. Examples of nonvolatile oils include, but are not limited to mono-, di-, and triesters.

Monoesters are defined as esters formed by the reaction of a monocarboxylic acid having the formula R-COOH, wherein R is a straight or branched chain saturated or unsaturated alkyl having 2 to 45 carbon atoms, or phenyl; and an alcohol having the formula R-OH wherein R is a straight or branched chain saturated or unsaturated alkyl having 2-30 carbon atoms, or phenyl. Both the alcohol and the acid may be substituted with one or more hydroxyl groups. Either one or both of the acid or alcohol may be a "fatty" acid or alcohol, and may have from about 6 to 30 carbon atoms, more preferably 12, 14, 16, 18, or 22 carbon atoms in straight or branched chain, saturated or unsaturated form. Examples of monoester oils that may be used in the compositions of the invention include hexyl laurate, butyl isostearate, hexadecyl isostearate, cetyl palmitate, isostearyl neopentanoate, stearyl heptanoate, isostearyl isononanoate, stearyl lactate, stearyl octanoate, stearyl stearate, isononyl isononanoate, and so on.

Suitable diesters are the reaction product of a dicarboxylic acid and an aliphatic or aromatic alcohol or an aliphatic or aromatic alcohol having at least two substituted hydroxyl groups and a monocarboxylic acid. The dicarboxylic acid may contain from 2 to 30 carbon atoms, and may be in the straight or branched chain, saturated or unsaturated form. The dicarboxylic acid may be substituted with one or more hydroxyl groups. The aliphatic or aromatic alcohol may also contain 2 to 30 carbon atoms, and may be in the straight or branched chain, saturated, or unsaturated form. Preferably, one or more of the acid or alcohol is a fatty acid or alcohol, i.e. contains 12-22 carbon atoms. The dicarboxylic acid may also be an alpha hydroxy acid. The ester may be in the dimer or trimer form. Examples of diester oils that may be used in the compositions of the invention include diisotearyl malate, neopentyl glycol dioctanoate, dibutyl sebacate, dicetearyl dimer dilinoleate, dicetyl adipate, diisocetyl adipate, diisononyl adipate, diisostearyl dimer dilinoleate, diisostearyl fumarate, diisostearyl malate, dioctyl malate, and so on.

Suitable triesters comprise the reaction product of a tricarboxylic acid and an aliphatic or aromatic alcohol or alternatively the reaction product of an aliphatic or aromatic alcohol having three or more substituted hydroxyl groups with a monocarboxylic acid. As with the mono- and diesters mentioned above, the acid and alcohol contain 2 to 30 carbon atoms, and may be saturated or unsaturated, straight or branched chain, and may be substituted with one or more hydroxyl groups. Preferably, one or more of the acid or alcohol is a fatty acid or alcohol containing 12 to 22 carbon atoms. Examples of triesters include esters of arachidonic, citric, or behenic acids, such as triarachidin, tributyl citrate, triisostearyl citrate, tri C₁₂₋₁₃ alkyl citrate, tricaprylin, tricaprylyl citrate, tridecyl behenate, trioctyldodecyl citrate, or tridecyl cocoate, tridecyl isononanoate, and so on.

Esters suitable for use in the composition are further described in the C.T.F.A. Cosmetic Ingredient Dictionary and Handbook, Eleventh Edition, 2006, under the classification of "Esters".

It may be desirable to incorporate one or more nonvolatile hydrocarbon oils into the composition. Suitable nonvolatile hydrocarbon oils include paraffinic hydrocarbons and olefins, preferably those having greater than about 20 carbon atoms. Examples of such hydrocarbon oils include C₂₄₋₂₈ olefins, C₃₀₋₄₅ olefins, C₂₀₋₄₀ isoparaffins, hydrogenated polyisobutene, polyisobutene, polydecene, hydrogenated polydecene, mineral oil, pentahydrosqualene, squalene, squalane, and mixtures thereof. In one preferred embodiment such hydrocarbons have a molecular weight ranging from about 300 to 1000 Daltons.

Synthetic or naturally occurring glyceryl esters of fatty acids, or triglycerides, are also suitable for use in the compositions. Both vegetable and animal sources may be used. Examples of such oils include castor oil, lanolin oil, C₁₀₋₁₈ triglycerides, caprylic/capric/triglycerides, sweet almond oil, apricot kernel oil, sesame oil, camelina sativa oil, tamanu seed oil, coconut oil, corn oil, cottonseed oil, linseed oil, ink oil, olive oil, palm oil, illipe butter, rapeseed oil, soybean oil, grapeseed oil, sunflower seed oil, walnut oil, and the like.

Also suitable are synthetic or semi-synthetic glyceryl esters, such as fatty acid mono-, di-, and triglycerides which are natural fats or oils that have been modified, for example, mono-, di- or triesters of polyols such as glycerin. In an example, a fatty (C₁₂₋₂₂) carboxylic acid is reacted with one or more repeating glyceryl groups. glyceryl stearate, diglyceryl diiosostearate, polyglyceryl-3 isostearate, polyglyceryl-4 isostearate, polyglyceryl-6 ricinoleate, glyceryl dioleate, glyceryl diisotearate, glyceryl tetraisostearate, glyceryl trioctanoate, diglyceryl distearate, glyceryl linoleate, glyceryl myristate, glyceryl isostearate, PEG castor oils, PEG glyceryl oleates, PEG glyceryl stearates, PEG glyceryl tallowates, and so on.

Nonvolatile silicone oils, both water soluble and water insoluble, are also suitable for use in the composition. Such silicones preferably have a viscosity ranging from about greater than 5 to 800,000 cst (5 × 10⁻⁶ m²/s to 0.8 m²/s), preferably 20 to 200,000 cst (20 × 10⁻⁶ m²/s to 0.2 m²/s) at 25°C. Suitable silicones include amine functional silicones such as amodimethicone dimethicone, phenyl dimethicone, diphenyl dimethicone, phenyl trimethicone, or trimethylsiloxyphenyl dimethicone. Other examples include alkyl dimethicones such as cetyl dimethicone, and the like wherein at least one R is a fatty alkyl (C₁₂, C₁₄, C₁₆, C₁₈, C₂₀, or C₂₂), and the other R is methyl, and A is a trimethylsiloxy endcap unit, provided such alkyl dimethicone is a pourable liquid at room temperature. Phenyl trimethicone can be purchased from Dow Corning Corporation under the tradename 556 Fluid. Trimethylsiloxyphenyl dimethicone can be purchased from Wacker-Chemie under the tradename PDM-1000. Cetyl dimethicone, also referred to as a liquid silicone wax, may be purchased from Dow Corning as Fluid 2502, or from DeGussa Care & Surface Specialties under the trade names Abil Wax 9801, or 9814.

The composition may contain one or more humectants. If present, they may range from about 0.01 to 75%, preferably from about 0.5 to 70%, more preferably from about 0.5 to 40%. Examples of suitable humectants include glycols, sugars, and the like. Suitable glycols are in monomeric or polymeric form and include polyethylene and polypropylene glycols such as PEG 4-10, which are polyethylene glycols having from 4 to 10 repeating ethylene oxide units; as well as C₁₋₆ alkylene glycols such as propylene glycol, butylene glycol, pentylene glycol, and the like. Suitable sugars, some of which are also polyhydric alcohols, are also suitable humectants. Examples of such sugars include glucose, fructose, honey, hydrogenated honey, inositol, maltose, mannitol, maltitol, sorbitol, sucrose, xylitol, xylose, and so on. Also suitable is urea. Preferably, the humectants used in the composition of the invention are C₁₋₆, preferably C₂₋₄ alkylene glycols, most particularly butylene glycol.

### B. Surfactants

It may be desirable for the composition to contain one more surfactants, especially if in the emulsion form. However, such surfactants may be used if the compositions are solutions, suspensions, or anhydrous also, and will assist in dispersing ingredients that have polarity, for example pigments. Such surfactants may be silicone or organic based. The surfactants will also aid in the formation of stable emulsions of either the water-in-oil or oil-in-water form. If present, the surfactant may range from about 0.001 to 30%, preferably from about 0.005 to 25%, more preferably from about 0.1 to 20% by weight of the total composition.

The composition may comprise one or more nonionic organic surfactants. Suitable nonionic surfactants include alkoxylated alcohols or ethers, formed by the reaction of an alcohol with an alkylene oxide, usually ethylene or propylene oxide. Suitable alcohols include mono-, di-, or polyhydric short chain (C1-6) alcohols; aromatic or aliphatic saturated or unsaturated fatty (C12-40) alcohols, of cholesterol; and so on.

In one embodiment the alcohol is cholesterol, or an aromatic or aliphatic saturated or unsaturated fatty alcohol which may have from 6 to 40, preferably from about 10 to 30, more preferably from about 12 to 22 carbon atoms. Examples include oleyl alcohol, cetearyl alcohol, cetyl alcohol, stearyl alcohol, isostearyl alcohol, behenyl alcohol, and the like. Examples of such ingredients include Oleth 2-100; Steareth 2-100; Beheneth 5-30; Ceteareth 2-100; Ceteth 2-100; Choleth 2-100 wherein the number range means the number of repeating ethylene oxide units, e.g. Ceteth 2-100 means Ceteth where the number of repeating ethylene oxide units ranges from 2 to 100. Derivatives of alkoxylated alcohols are also suitable, such as phosphoric acid esters thereof.

Some preferred organic nonionic surfactants include Oleth-3, Oleth-5, Oleth-3 phosphate, Choleth-24; Ceteth-24; and so on.

Also suitable are alkoxylated alcohols formed with mono-, di-, or polyhydric short chain alcohols, for example those having from about 1 to 6 carbon atoms. Examples include glucose, glycerin, or alkylated derivatives thereof. Examples include glycereth 2-100; gluceth 2-100; methyl gluceth 2-100 and so on. More preferred are methyl gluceth-20; glycereth-26 and the like.

Other types of alkoxylated alcohols are suitable surfactants, including ethylene oxide polymers having varying numbers of repeating EO groups, generally referred to as PEG 12 to 200. More preferred are PEG-75, which is may be purchased from Dow Chemical under the trade name Carbowax PEG-3350.

Other suitable nonionic surfactants include alkoxylated sorbitan and alkoxylated sorbitan derivatives. For example, alkoxylation, in particular ethoxylation of sorbitan provides polyalkoxylated sorbitan derivatives. Esterification of polyalkoxylated sorbitan provides sorbitan esters such as the polysorbates. For example, the polyalkyoxylated sorbitan can be esterified with C6-30, preferably C12-22 fatty acids. Examples of such ingredients include Polysorbates 20-85, sorbitan oleate, sorbitan sesquioleate, sorbitan palmitate, sorbitan sesquiisostearate, sorbitan stearate, and so on.

Also suitable are various types of silicone or silane-based surfactants. Examples include organosiloxanes substituted with ethylene oxide or propylene oxide groups such as PEG dimethicones which are dimethicones substituted with polyethylene glycols including those having the INCI names PEG-1 dimethicone; PEG-4 dimethicone; PEG-8 dimethicone; PEG-12 dimethicone; PEG-20 dimethicone; and so on.

Also suitable are silanes substituted with ethoxy groups or propoxy groups or both, such as various types of PEG methyl ether silanes such as bis-PEG-18 methyl ether dimethyl silane; and so on.

Further examples of silicone based surfactants include those having the generic names dimethicone copolyol; cetyl dimethicone copolyol; and so on.

The topical composition may also contain a variety of other ingredients including but not limited to preservatives, pH adjusters, and the like.

### Examples of topical compositions include:

A water and oil emulsion containing:
0.01 to 10% of the sinapoyl malate,
0.1 to 90% water,
0.1 to 40% oil, preferably silicone oils or esters,
0.1 to 10% surfactant, preferably a non-ionic surfactant.

The composition may be in the form of a cream, lotion, or serum. The composition may also be in the form of a color cosmetic composition such as foundation makeup, concealer, primer, eye shadow, and the like.

### III. The Methods

The invention comprises a composition for use in methods for treating skin by topically applying the topical composition comprising a synthetic ester of sinapinic acid that is sinapoyl malate to inhibit UV damage in skin cells, as an anti-oxidant to reduce or inhibit free radicals on skin, to reduce or inhibit DNA damage in skin cells exposed to UV light, and as an anti-inflammatory to reduce skin irritation, inflammation and sensitivity.

The invention is also directed to a composition of the invention for use in a method for improving cellular health and viability by topically applying a composition comprising an effective amount of sinapoyl malate.

The invention is also directed to a composition of the invention for use in a method for inhibiting and/or repairing DNA damage in skin cells by topically applying a composition comprising an effective amount of sinapoyl malate.

The invention is also directed to a composition of the invention for use in a method for reducing skin irritation or inflammation by applying a topical composition comprising an effective amount of sinapoyl malate. More preferred is where the sinapoyl malate inhibits irritation or inflammation by reducing or inhibiting product of IL1-α and/or IL1-β in skin cells.

The invention is also directed to a composition of the invention for use in a method for scavenging free radicals on skin by topically applying a composition comprising an effective amount of sinapoyl malate.

The desired benefits may be obtained by applying topical compositions containing the Sinapoyl malate in effective amounts to the skin. The types of compositions applied may be as set forth herein. The compositions may be applied in regimens, or in the morning or evening or at other times of the day.

### IV. The Synthetic Method

The invention is also directed to a method for making a topical composition comprising synthesizing sinapoyl malate and then formulating the sinapoyl malate into a topical composition; wherein the synthesis of the sinapoyl malate comprises the steps of:
(a) (i) reacting syringaldehyde with acetic acid anhydride and an alkali metal acetate; or (ii) reacting sinapinic acid with acetic acid anhydride,
(b) cleaving the intermediate obtained in section (a) by exposing to water and an aliphatic alcohol to form protected sinapinic acid,
(c) esterifying the protected sinapinic acid by reacting with an alkyl protected carboxylic acid ester to form protected sinapoyl malate,
(d) reacting the protected sinapoyl malate with aqueous acid to yield sinapoyl malate.

Preferred is where the starting material is syringaldehyde which is reacted with an alkali metal acetate (such as sodium), alkanol (methanol, propanol, butanol), and acetic anhydride. The resulting acetyl protected sinapinic acid may then be esterified by reacting with a malic acid ester CO₂R-CH₂CH(OH)-CO₂R where R may be selected from methyl, ethyl, propyl, iso-propyl or butyl, to yield sinapinic acid ester where all hydroxyl groups are protected. The protecting groups can be hydrolyzed by reacting with aqueous inorganic acids such as hydrochloric or sulfuric acid and an appropriate organic solvent to yield sinapoyl malate.

The invention will be further described in connection with the following examples which are set forth for the purposes of illustration only.

### EXAMPLE 1

Sinapoyl malate was prepared as follows:

### Step 1: Synthesis of 4-acetoxy-sinapinic acid, S3.

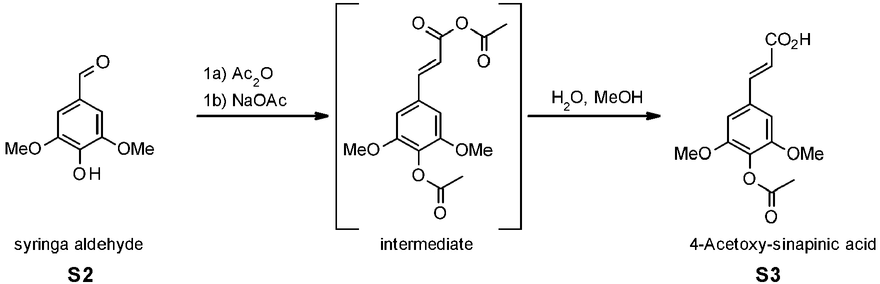

In a 1 liter flask a reaction mixture of syringealdehyde (150 grams, 0.82 mol), acetic acid anhydride (293 grams, 2.87 mol) and sodium acetate (59 grams, 0.72 mol) was preheated to 80° C. for 5 hours before heating to reflux for an additional 8 hours. Afterwards the atmosphere was lowered to 500 mbar (50 kPa) and acetic acid was removed from the mixture. The residue was cooled to 90° C. and methanol (900 grams) was added followed by addition of water (240 grams) at room temperature (25° C.). The reaction mixture was stirred thoroughly for 3 hours at this temperature. Afterwards, the methanol was removed *in vacuo.* To the obtained residue, toluene (750 grams) was added and the organic and aqueous layers were separated yielding the raw product in toluene. The toluene was removed in vacuo until the product started to precipitate. The reaction mixture was cooled to 0° C. and stirred at this temperature for an additional hour. The precipitate was removed by filtration and washed with cold toluene. Yield of product was 76 grams (35%), and spectroscopic data confirmed the correct compound.

### Step 2: Synthesis of 4-acetoxy-sinapoyl-di-isopropyl malate, S5.

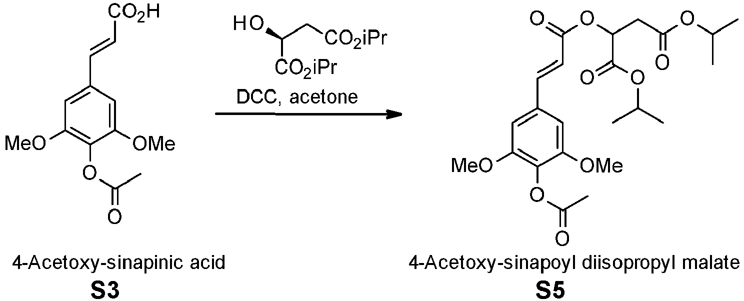

In a 1 liter flask with 4-acetoxy-sinapinic acid S3 (40 grams, 0.15 mol), freshly distilled diisopropyl malate (32.7 grams, 0.15 mol) and acetone (415 grams), a solution of dicyclohexyl carbodiimide (34 grams, 0.165 mol), 4-dimethyl aminopyridine (DMAP, 7.4 grams, 0.06 mol) in acetone (150 grams) was added dropwise at room temperature. The resulting reaction mixture was stirred for 5 hours at this temperature. The precipitate was removed by filtration, washed with acetone and the solvent removed *in vacuo.* The obrained residue was dissolved in methyl-tert-butyl ether (200 grams), removed *in vacuo* to yield the desired raw product S5 (83 grams) which was used directly for the next reaction step without further purification. The spectroscopic data confirmed the desired compound.

### Step 3: Synthesis of Sinapoyl malate, S6.

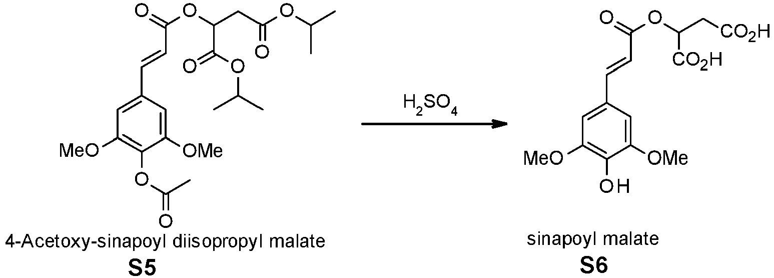

To a 2 liter flask containing 4-acetoxy sinapoyl-di-isopropyl malate S5 (83 grams) in acetone (750 grams) was added aqueous sulfuric acid (40%, 290 grams). The mixture was heated at reflux conditions for 8 hours until deprotection was completed. Under partial vacuum (700 mbar) acetone was evaporated from the mixture yielding an aqueous residue. Ethyl acetate (600 grams) was added and the layers were separated, the organic layer washed with water and the solvent removed *in vacuo* to yield a brown residue. The raw product was dissolved in methyl-tert-butyl ether (350 grams) and extracted with saturated NaHCO3 solution, which was then acidified with H2SO4 and extracted with methyl-tert-butyl ether afterwards yielding the raw product after evaporating the organic layers to dryness. Sinapoyl malate was obtained as a light yellow solid (10 grams, 20% two steps) after flash column chromatography (cyclohexane:ethyl acetate, 1:1); melting range 55-65° C., 1H NMR (400 MHz, DMSO-d6): δ 9.00 (s, 1H), 7.59 (d, J=15.8 Hz, 1H), 7.06 (s, 2H), 6.59 (d, J=15.9 Hz), 5.33 (dd, J=8.8, 3.9 Hz, 1H), 3.86-3.77 (m, 6H), 2.89 (dd, J=16.7, 3.9 Hz, 1H), 2.77 (dd, J=16.7, 8.8 Hz, 1H). 13C NMR (101 MHz, DMSO-d6): 170.61, 170.27, 165.71, 147.90, 146.36, 138.40, 124.12, 113.89, 106.29, 68.28, 55.99, 40.02, 39.81, 39.60, 39.39, 39,18, 38.98, 38.77, 35.81; HR-LCMS (ESI) m/z 339.0717 (M-H+), 4.49 min; HPLC 4.67 min.

All HPLC chromatograms were recorded on an HPLC system using a poroshell 120 SB-C18 column (2.7 um, 50.x2.1 mm) with a gradient 5% to 50% (10 min) and 50-100% (2 min) water (0.1% formic acid) and acetonitrile as eluent and a flow of 0.40 ml/min at 40° C. As detector a DAD UV 220-320 nm was used. All LCMS chromatograms were recorded on an HPLC System using a Kinetex RP-C18 column (1.7 um, 100 × 2.1 mm) with a gradient 0-95% (22 min) water (0.1% formic acid) and acetonitrile (0.09% formic acid) as eluent and a flow of 0.55 ml/min at 50o C. As MS detector a Bruker micrOTOF Q-11 was used.

### EXAMPLE 2

The sinapinic acid ester (sinapoyl malate) prepared in Example 1 having the structure: 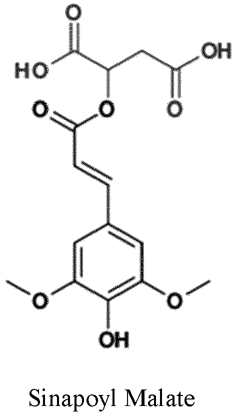 was comparatively tested against caftaric acid phenethyl ester (CAPE) having the following structure: 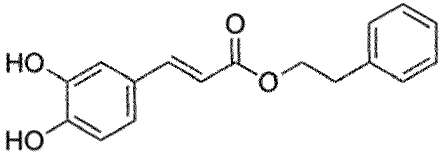 Sinapoyl malate and CAPE were evaluated for UV absorption. 50 and 100 ppm of each compound was dissolved in water and a second sample set of isopropanol. Additional samples of sinapoyl malate and CAPE were prepared by dissolving 0.025% and 0.05% of each ingredient in a 50/50 mixture of water and butylene glycol. Further dilution to obtain a concentration of 1.4 ppm and 14 ppm was performed.

The samples were tested for UV absorption across the UV range by measuring with an Agilent 8453 UV visible spectroscopy system at wavelengths ranging across 200 to 450 nm at time 0, week 1, week 2, and week 4. The term "NA" means that a previously observed absorbance peak was no longer visible. The term "peak" means the two highest absorbance peaks in the UV spectrum. The term "valley" means the two lowest point between the absorbance peaks. In the case where there is only one peak or valley it means that the other, previously observed peak or valley was deteriorated at the time and temperature of the stability study. In the case where all peaks and valleys are indicated "NA" it means that the sample deteriorated entirely and no longer absorbed in the UV range at all.

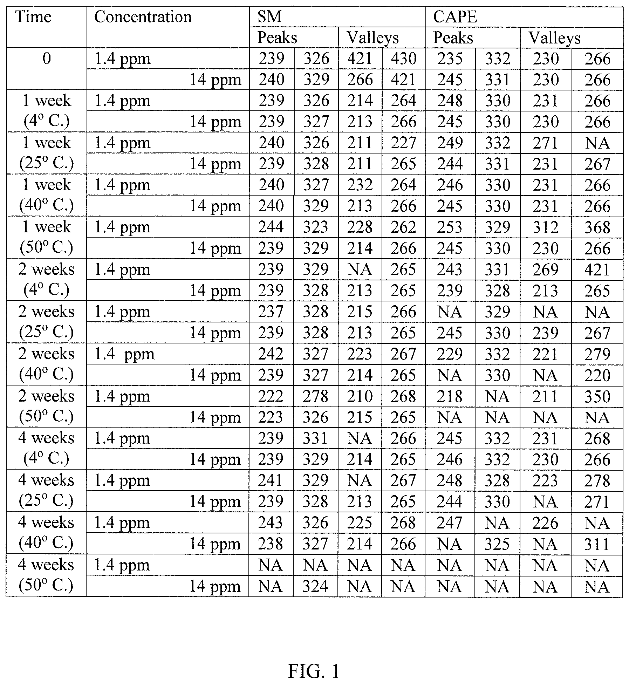

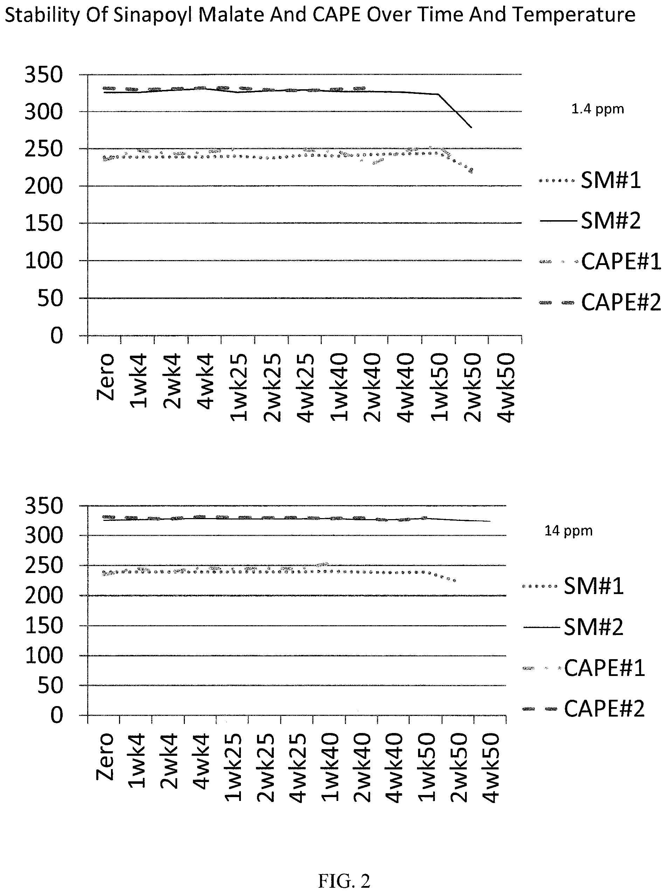

The numerical results are set forth in Fig. 1 and graphically depicted in Fig. 2. It is seen that the absorbance peaks of Sinapoyl malate ("S") are different and distinct from the absorbance peaks of CAPE ("C"). In addition, Sinapoyl malate is more stable over time and temperature at concentrations of 1.4 and 14 ppm. In contrast, CAPE shows much less stability over time and temperature. Accordingly, the difference in chemical structure between the two compounds demonstrates unexpected differences in both UV absorbance peaks and valleys as well as long term stability and show that when the absorbance peaks in the UV range were measured on samples containing sinapoyl malate ("SM") and CAPE dissolved in a 50/50 mixture of water and butylene glycol with final concentration at 1.4 and 14 ppm, SM exhibited different absorbance peaks than CAPE and showed significantly improved overall stability than CAPE over time and temperature. This is unexpected given the similarity in structures between the two compounds. The term "NA" means that the previously observed absorbance peak from the pair was no longer visible or measurable.

### EXAMPLE 3

The sinapoyl malate prepared in Example 1 was tested for impact on cellular health and viability at concentrations ranging from 0, 0.0025, 0.005, 0.0075, 0.01, 0.025, 0.05, 0.075, 0.1 and 0.25% on normal human epidermal keratinocytes (NHEK) that were not irradiated, and that were irradiated with 20, 40, and 80 mJ/cm² UVB using the Almar Blue assay.

More specifically, normal human dermal keratinocytes were harvested and assayed for cellular viability when untreated or treated with sinapoyl malate at the above mentioned concentrations.

The cells were placed in concentrations of 150,000 cells per plate for the 48 hour test, and 300,000 cells per plate for the 24 hour test on 96 well plates. The cells were incubated at 37° C., 5% CO₂, and 95% humidity for 24 hours. The test compositions were prepared as follows:
Cells were treated for 48 hours by applying the test compositions. The cells were kept in an incubator with conditions as set forth above.

After 48 hours the cells were washed with DPBS and covered with a thin layer (about 100 µl) of DPBS. The DPBS was removed and then 100 µl of the test compositions was placed on the cells for 24 hours. The cells were kept in the incubator with the conditions as set forth herein.

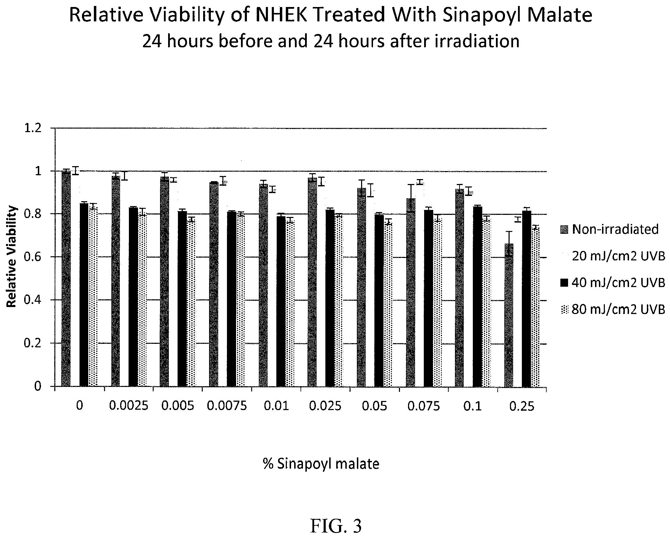

Other batches of cells were irradiated with 20, 40, and 80 mJ/cm² UVB (Dr. Groebel, UV-Electronik, GmbH). The DPBS was aspirated and the treatment compositions applied once again for 24 hours. The next morning the medium was aspirated and 100 µl of 10% Alamar Blue solution was added. The plate was incubated at 37° C. for 1.5 to 2 hours. The fluorescence was measured at 530/590 nanometers using a Spectra Max Gemini reader. The cell viability was calculated and expressed as the percentage of survival of cells treated with hydrogen peroxide. The results are set forth in Fig. 3 and demonstrate that Sinapoyl malate was effective in promoting keratinocyte health and viability both before and after UV radiation.

### EXAMPLE 4

Sinapoyl malate was tested in the DNA fragmentation study to ascertain its ability to repair or inhibit DNA fragmentation upon exposure of cells to UV radiation. Keratinocytes were plated in 60mm dishes at 100,000 cells per dish and grown in EpiLife medium (ThermoFisher, cat#M-EPI-500-CA) supplemented with Human Keratinocyte Growth Supplement (ThermoFisher, cat#S-001-5) until they reached 50% confluency. Cells were washed with DPBS and then covered with a thin layer (2 mL) of DPBS containing 0.005% or 0.025% of Sinapoyl malate before being irradiated with 100 mJ/cm² UVB in the Dr. Gröbel irradiation chamber. Cells were washed again with DPBS and incubated in medium for 6h. NHEK were trypsinized, washed with PBS and suspended in PBS at 1×10⁵ cells/mL. Cells were then dispersed in melted agarose (Trevigen, cat#4250-050-02) at 37°C at a 1:10 ratio. 75µl of the cell/agarose mixture was pipetted evenly on to each spot of the comet slide (Trevigen, cat#4250-050-03) and then incubated at 4°C for 10 minutes. Slides were immersed in cold lysis solution (Trevigen, cat#4250-050-01) on ice for overnight. Slides were removed from the lysis solution and placed into an alkaline solution (300mM NaOH, ImM EDTA, pH>13) at room temperature for 30 minutes. Then the slides were placed in the Comet Assay ESII Electrophoresis System. Cold alkaline electrophoresis solution (200mM NaOH, ImM EDTA, pH>13) was poured into the apparatus so that it just covered the slides. Electrophoresis ran for 30 minutes at 23V. After electrophoresis the slides were rinsed in H₂O and immersed in 70% EtOH for 5 min. Slides were removed from the EtOh solution and placed on a towel to air dry overnight. SYBR gold (ThermoFisher, cat#S11494) was diluted in TE buffer (10 mM Tris-HCl, ImM EDTA, pH 7.5) 1:30000. 100 µl of diluted SYBR gold was pipetted on to each spot. Slides were incubated at room temperature for 30 min. Then slides were allowed to dry again after removing excess SYBR gold from the slides. Slides were viewed under the EVOS microscope with the FITC filter with the 20x objective. The tail moments were determined with the Comet Score software from Tri Tek.

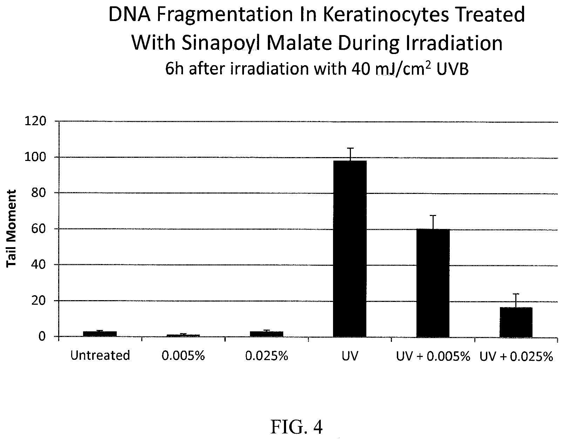

The results are set forth in Fig. 4 and show that Sinapoyl malate was a very effective in inhibiting or preventing DNA damage in keratinocytes both in cells treated with a concentration of 0.005% and 0.025% Sinapoyl malate and no irradiation (left side of graph) and cells irradiated with UV and exposed to 0.005% and 0.025% Sinapoyl malate.

### EXAMPLE 5

The anti-inflammatory activity of Sinapoyl malate was demonstrated by testing its ability to inhibit Interleukin-la (IL1-α) and IL1-β which are both indicators of inflammation. IL1 species are responsible for stimulating inflammation in damaged tissues.

Keratinocytes were treated as described in Example 4. The media that the cells were incubated in following irradiation was collected after 6h and analyzed with the Milliplex _{MAP} Human Cytokine Assay (Millipore, cat#HCYTMAG-60K-PX29) according to the manufacturer's protocol. Media from the cells were incubated with pre-mixed magnetic beads overnight at 4°C on a shaker at 800 rpm. Beads were washed and then incubated with the detection antibodies for 1h at room temperature on the shaker at 800rpm. Streptavidin-Phycoerythrin was added to the beads and incubated for 30 min at room temperature on the shaker at 800rpm. Beads were washed, suspended in sheath fluid and read on the Luminex.

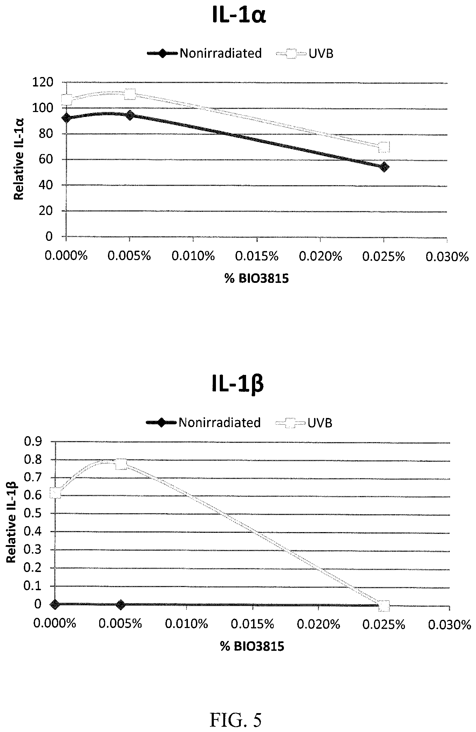

The results are set forth in Fig. 5 with "BIO3815" referring to Sinapoyl malate. The results demonstrate that Sinapoyl malate inhibits both IL1-α and IL1-β in a dose dependent manner thus showing its effectiveness as an anti-inflammatory active.

### EXAMPLE 6

The anti-oxidant activity of Sinapoyl malate was tested by first determining acceptable non-cytotoxic concentrations of this material. Based on these results, Normal Human Epidermal Keratinocyte (NHEK) were plated on two 96-well microtiter plates at a titer of 2 × 10⁴ cells per well. In addition to untreated control samples, Sinapoyl malate was added at concentrations that ranged from 0.01% to 0.1% and incubated overnight (ca. 15 h). The following day, cell culture media were aspirated, the cells were rinsed with 100 ul of Dulbecco's Phosphate Buffered Saline, pH 7.4 (D-PBS), aspirated again and then 30 ul D-PBS was added. One plate was kept in the cell culture hood, as a sham-irradiated sample, while the other plate was exposed to 50 mJ/cm² UVB. After exposure and further aspiration, the Control and the UVB-irradiated plates were treated with 100 ul of 10 uM 2',7'-dichlorodihydrofluorescein diacetate (DCFda) in D-PBS for 6h at 37 C°. In addition, 100 ul of 25 mM NaN₃ was added 20 minutes into the incubation. At the end of the 6h incubation, fluorescence was measured with a SpectraMax Gemini EM fluorescence plate reader set at 485 nm excitation and 538 nm emission with a wavelength cut off set at 530 nm. An increase in fluorescence indicates increased reactive oxygen species (ROS), particularly hydrogen peroxide (H₂O₂) because it is the longest lived ROS. Conversely, a reduction in fluorescence indicates antioxidant activity. In this way, both endogenous and UVB-induced ROS were determined and the effectiveness of Sinapoyl malate as an antioxidant measured.

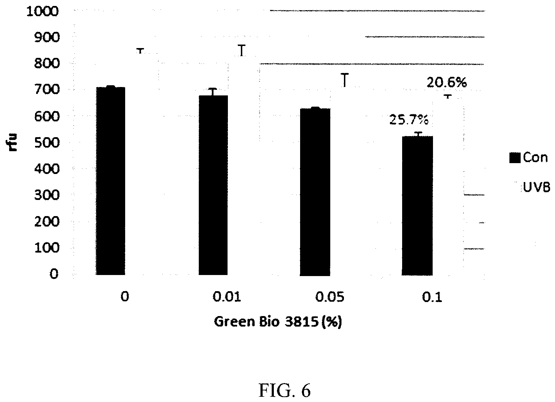

The results are set forth in Fig. 6 and show that Sinapoyl malate was a very effective anti-oxidant for both untreated cells and cells irradiated with UV at concentrations of 0.01%, 0.05%, and 0.10%.

## Claims

1. A topical composition comprising a synthetic ester of sinapinic acid that is sinapoyl malate.

2. The composition of claim 1 in the form of a cream, lotion, serum, gel, solution, suspension, or anhydrous composition.

3. The composition of claim 2 in the form of a water and oil emulsion.

4. A composition for use in a method for treating skin to provide one or more benefits selected from (a) protecting against UV radiation, (b) inhibiting DNA damage in skin cells, (c) inhibiting or reducing skin inflammation, and (d) scavenging free radicals by topically applying the composition; wherein the composition comprises a synthetic ester of sinapinic acid that is sinapoyl malate.

5. The composition for use according to claim 4 wherein the skin is protected against UVA and UVB radiation.

6. The composition for use according to claim 4 wherein DNA damage in skin cells is inhibited.

7. The composition for use according to claim 4 wherein inflammation is inhibited by inhibiting IL1-α or IL1-β or both.

8. The composition for use according to claim 7 wherein the sinapoyl malate is at a concentration of 0.001 to 0.05%.

9. The composition for use according to claim 4 wherein the sinapoyl malate scavenges free radicals when topically applied to skin.

10. The composition for use according to claim 4 wherein the sinapoyl malate reduces or inhibits DNA damage in skin cells when topically applied.

11. The composition for use according to claim 4 wherein the sinapoyl malate protects the skin cells against UV radiation when topically applied.

12. A method for making a topical composition comprising synthesizing sinapoyl malate and then formulating the sinapoyl malate into a topical composition;
wherein the synthesis of the sinapoyl malate comprises the steps of:
(a)
(i) reacting syringaldehyde with acetic acid anhydride and an alkali metal acetate;
or (ii) reacting sinapinic acid with acetic acid anhydride,
(b) cleaving the anhydride of the acetyl protected reaction product of (a) by exposing to water and an aliphatic alcohol to form protected sinapinic acid,
(c) esterifying the protected sinapinic acid by reacting with an alkyl protected carboxylic acid ester to form protected sinapoyl malate,
(d) reacting the protected sinapoyl malate with aqueous acid to yield sinapoyl malate.

13. The method of claim 12 wherein step (a) is (a)(i).

14. The method of claim 13 wherein the syringaldehyde is reacted with sodium acetate and acetic anhydride, or
wherein the aliphatic alcohol in (b) is methanol; the alkyl protected carboxylic acid ester in (c) is an ester of malic acid, and the aqueous acid in (d) is hydrochloric acid, sulfuric acid or combinations thereof.

15. The method of any one of claims 12-14, wherein the alkyl protected carboxylic acid ester in (c) is a di-isopropyl ester.

## Patentansprüche

1. Topische Zusammensetzung, umfassend einen synthetischen Ester von Sinapinsäure, der Sinapoylmalat ist.

2. Zusammensetzung nach Anspruch 1 in Form einer Creme, Lotion, eines Serums, Gels, einer Lösung, Suspension oder wasserfreien Zusammensetzung.

3. Zusammensetzung nach Anspruch 2 in Form einer Wasser-Öl-Emulsion.

4. Zusammensetzung zur Verwendung in einem Verfahren zur Behandlung von Haut, um einen oder mehrere Vorteile bereitzustellen, ausgewählt aus (a) Schützen vor UV-Strahlung, (b) Hemmen von DNA-Schäden in Hautzellen, (c) Hemmen oder Reduzieren von Hautentzündungen und (d) Abfangen von freien Radikalen durch topisches Auftragen der Zusammensetzung;
wobei die Zusammensetzung einen synthetischen Ester von Sinapinsäure umfasst, der Sinapoylmalat ist.

5. Zusammensetzung zur Verwendung nach Anspruch 4, wobei die Haut gegen UVA- und UVB-Strahlung geschützt ist.

6. Zusammensetzung zur Verwendung nach Anspruch 4, wobei DNA-Schäden in Hautzellen gehemmt werden.

7. Zusammensetzung zur Verwendung nach Anspruch 4, wobei die Entzündung durch Hemmen von IL1-α oder IL1-β oder beidem gehemmt wird.

8. Zusammensetzung zur Verwendung nach Anspruch 7, wobei das Sinapoylmalat in einer Konzentration von 0,001 bis 0,05 % vorliegt.

9. Zusammensetzung zur Verwendung nach Anspruch 4, wobei das Sinapoylmalat bei topischer Anwendung freie Radikale abfängt.

10. Zusammensetzung zur Verwendung nach Anspruch 4, wobei das Sinapoylmalat bei topischer Anwendung DNA-Schäden in Hautzellen reduziert oder hemmt.

11. Zusammensetzung zur Verwendung nach Anspruch 4, wobei das Sinapoylmalat die Hautzellen bei topischer Anwendung vor UV-Strahlung schützt.

12. Verfahren zur Herstellung einer topischen Zusammensetzung, umfassend das Synthetisieren von Sinapoylmalat und dann das Formulieren des Sinapoylmalats zu einer topischen Zusammensetzung;
wobei die Synthese des Sinapoylmalats die folgenden Schritte umfasst:
(a) (i) Umsetzen von Syringaldehyd mit Essigsäureanhydrid und einem Alkalimetallacetat; oder (ii) Umsetzen von Sinapinsäure mit Essigsäureanhydrid,
(b) Spalten des Anhydrids des acetylgeschützten Reaktionsprodukts von (a) durch Aussetzen gegenüber Wasser und einem aliphatischen Alkohol, um geschützte Sinapinsäure zu bilden,
(c) Verestern der geschützten Sinapinsäure durch Umsetzen mit einem alkylgeschützten Carbonsäureester, um geschütztes Sinapoylmalat zu bilden,
(d) Umsetzen des geschützten Sinapoylmalats mit wässriger Säure, um Sinapoylmalat zu ergeben.

13. Verfahren nach Anspruch 12, wobei Schritt (a) (a)(i) ist.

14. Verfahren nach Anspruch 13, wobei der Syringaldehyd mit Natriumacetat und Essigsäureanhydrid umgesetzt wird, oder
wobei der aliphatische Alkohol in (b) Methanol ist; der alkylgeschützte Carbonsäureester in (c) ein Ester von Apfelsäure ist und die wässrige Säure in (d) Salzsäure, Schwefelsäure oder Kombinationen davon ist.

15. Verfahren nach einem der Ansprüche 12 bis 14, wobei der alkylgeschützte Carbonsäureester in (c) ein Diisopropylester ist.

## Revendications

1. Composition topique comprenant un ester synthétique d'acide sinapique qui est le malate de sinapoyle.

2. Composition selon la revendication 1 sous la forme d'une crème, d'une lotion, d'un sérum, d'un gel, d'une solution, d'une suspension ou d'une composition anhydre.

3. Composition selon la revendication 2 sous la forme d'une émulsion d'eau et d'huile.

4. Composition destinée à être utilisée dans un procédé de traitement de la peau pour fournir un ou plusieurs bénéfices choisis parmi (a) une protection contre les rayons UV, (b) une inhibition des lésions de l'ADN dans les cellules cutanées, (c) une inhibition ou une diminution de l'inflammation de la peau et (d) une récupération de radicaux libres par application topique de la composition ; dans laquelle la composition comprend un ester synthétique d'acide sinapique qui est le malate de sinapoyle.

5. Composition destinée à être utilisée selon la revendication 4 dans laquelle la peau est protégée contre les rayons UVA et UVB.

6. Composition destinée à être utilisée selon la revendication 4 dans laquelle les lésions de l'ADN dans les cellules cutanées sont inhibées.

7. Composition destinée à être utilisée selon la revendication 4 dans laquelle l'inflammation est inhibée en inhibant l'IL1-α ou l'IL1-β ou les deux.

8. Composition destinée à être utilisée selon la revendication 7 dans laquelle le malate de sinapoyle présente une concentration allant de 0,001 à 0,05 %.

9. Composition destinée à être utilisée selon la revendication 4 dans laquelle le malate de sinapoyle récupère des radicaux libres lorsqu'il est appliqué par voie topique sur la peau.

10. Composition destinée à être utilisée selon la revendication 4 dans laquelle le malate de sinapoyle réduit ou inhibe les lésions de l'ADN dans les cellules cutanées lorsqu'il est appliqué par voie topique.

11. Composition destinée à être utilisée selon la revendication 4 dans laquelle le malate de sinapoyle protège les cellules cutanées contre les rayons UV lorsqu'il est appliqué par voie topique.

12. Procédé de fabrication d'une composition topique comprenant la synthèse du malate de sinapoyle, puis la formulation du malate de sinapoyle dans une composition topique ;
dans lequel la synthèse du malate de sinapoyle comprend les étapes consistant à :
(a) (i) faire réagir du syringaldéhyde avec de l'anhydride d'acide acétique et un acétate de métal alcalin ; ou (ii) faire réagir un acide sinapique avec de l'anhydride d'acide acétique,
(b) cliver l'anhydride du produit de réaction protégé par l'acétyle de (a) en l'exposant à de l'eau et à un alcool aliphatique pour former de l'acide sinapique protégé,
(c) estérifier l'acide sinapique protégé en le faisant réagir avec un ester d'acide carboxylique protégé par un alkyle pour former du malate de sinapoyle protégé,
(d) faire réagir le malate de sinapoyle protégé avec un acide aqueux pour produire du malate de sinapoyle.

13. Procédé selon la revendication 12 dans lequel l'étape (a) est (a)(i).

14. Procédé selon la revendication 13 dans lequel le syringaldéhyde est mis en réaction avec l'acétate de sodium et l'anhydride acétique, ou
dans lequel l'alcool aliphatique dans (b) est le méthanol ; l'ester d'acide carboxylique protégé par un alkyle dans (c) est un ester d'acide malique, et l'acide aqueux dans (d) est l'acide chlorhydrique, l'acide sulfurique ou des combinaisons de ceux-ci.

15. Procédé selon l'une quelconque des revendications 12-14, dans lequel l'ester d'acide carboxylique protégé par un alkyle dans (c) est un ester diisopropylique.
